# FASCICULE DE BREVET EUROPEEN

(11) **EP 3 440 236 B1**
(45) Date de publication et mention de la délivrance du brevet: **11.06.2025**
(21) Numéro de dépôt: 17720546.5
(22) Date de dépôt: 05.04.2017
(51) Int. Cl.: C23F 1/26, C23F 1/38, A61C 13/00, B24C 1/00, B24C 11/00, A61C 8/00, A61L 27/06, A61L 27/50, A61L 27/56

(54) **PROCÉDÉ DE TRAITEMENT DE SURFACE D'UN MATÉRIAU MÉTALLIQUE BIOCOMPATIBLE ET IMPLANT TRAITÉ PAR LEDIT PROCÉDÉ**
VERFAHREN ZUR BEHANDLUNG VON OBERFLÄCHEN METALLISCHER BIOKOMPATIBLER MATERIALIEN UND DAMIT BEHANDELTES IMPLANTAT
PROCESS OF TREATING A SURFACE OF A METALLIC BIOCOMPATIBLE MATERIAL AND IMPLANT TREATED BY SAID PROCESS

(30) Priorité: 08.04.2016 FR 1653094
(43) Date de publication de la demande: 13.02.2019
(73) Titulaire: Selenium Medical, 17000 La Rochelle (FR)
(72) Inventeur: RICHART, Olivier, 17140 Lagord (FR); DEPERY, Hervé, 74940 Annecy Le Vieux (FR)
(74) Mandataire: Ipsilon
(86) Numéro de dépôt international: PCT/FR2017/050795
(87) Numéro de publication internationale: WO 2017/174924

(56) Documents cités:
- EP-A1- 1 847 278
- EP-A1- 1 847 278
- EP-A1- 2 476 443
- EP-A1- 2 476 443
- WO-A1-2013/124693
- WO-A1-2013/124693
- CN-A- 104 451 684
- CN-A- 104 451 684
- FR-A1- 2 906 147
- FR-A1- 2 906 147
- US-A1- 2011 233 169
- US-A1- 2011 233 169

## Description

### DOMAINE DE L'INVENTION

La présente invention concerne le domaine des matériaux biocompatibles, et plus particulièrement le domaine des implants, notamment les implants dentaires ou rachidiens

Elle concerne en particulier un procédé de la préparation et du traitement de la surface d'un tel matériau.

### ART ANTERIEUR

A l'heure actuelle, les implants dentaires, dispositifs destinés à être partiellement insérés dans la mâchoire d'un patient en vue de remplacer une dent, sont généralement réalisés à base d'alliages métalliques. Ces alliages renferment le plus souvent du titane, pour des raisons de résistance. Il en est de même pour les implants rachidiens.

En vue de favoriser l'ostéo-intégration d'un implant, ont été proposés soit l'application de revêtements comprenant du phosphate calcique (sous forme par exemple d'hydroxyapatite, de nature chimique proche de celle de l'os) soit des traitements de surface pour augmenter à la fois leur rugosité de surface et leur caractère hydrophile.

Les traitements de surface actuels comprennent le plus souvent un sablage de ladite surface au moyen de grains d'alumine, et/ou un traitement par une solution acide (acides inorganiques tels que les acides fluorhydrique, chlorhydrique, sulfurique, phosphorique, nitrique...ou un mélange de plusieurs de ces acides).

Un alliage à base de titane, d'aluminium et de vanadium, à usage médical a été récemment mis dans le commerce : il s'agit du TA6V ELI (Extra Low Interstitial).

Cependant les traitements actuels de surface de cet alliage TA6V ELl, destinés à créer une macroporosité et une microporosité en surface, ne sont pas satisfaisants pour les raisons suivantes :
- Pour créer la macroporosité de surface, les traitements de sablage avec des particules d'alumine (particules bon marché et très dures) laissent des microbilles d'alumine incrustées à la surface du matériau,
- Un traitement ultérieur à l'acide fluorhydrique peut les éliminer, mais fragilise le matériau et dégrade ses propriétés mécaniques ;
- Pour créer la microporosité sont ensuite utilisées des attaques acides au moyen de solutions d'acide chlorhydrique, et/ou des solutions associant l'acide fluorhydrique et l'acide nitrique, qui attaquent la surface du matériau en laissant saillants ou désincrustant des atomes de Vanadium.

Ni la présence d'impuretés d'alumine, ni les atomes de vanadium en surface ne sont souhaitées par les praticiens. En effet dans le domaine dentaire chaque impureté peut être une cause d'échec de l'ostéo-intégration d'un implant.

Ainsi l'inconvénient de l'alliage TA6V ELl est la présence de vanadium (4% en poids) et d'aluminium (6% en poids) dans sa composition. Ces deux éléments, qui ne sont pas biocompatibles, peuvent altérer les propriétés biologiques des implants réalisés dans ce matériau.

WO2013/124693 décrit un procédé de fabrication d'implants superhydrophiles. US2011/233169 décrit un implant dentaire présentant une topographie de surface à l'échelle nanométrique et un procédé de fabrication d'un tel implant dentaire. CN104451684 décrit un procédé de fabrication d'une surface multifonctionnelle d'un implant bionique à base de titane. EP1847278 décrit un implant dentaire, un emballage pour cet implant dentaire et un un procédé de traitement de cet implant dentaire, qui que la surface biologiquement active de l'implant ne soit altérée par des contaminants. EP2476443 décrit un implant dentaire à base de céramique présentant une surface hydrophile pour une insertion au moins partielle dans un os.

### BUTS DE L'INVENTION

Un premier but de la présente invention est donc de pallier les inconvénients des procédés ci-dessus et de proposer un procédé de traitement de surface d'un matériau métallique biocompatible, tel qu'un implant, qui augmente la rugosité de surface sans laisser de résidus pouvant freiner l'ostéo-intégration dudit matériau.

Un autre but de l'invention est de proposer un procédé de traitement de surface qui soit adaptable à l'alliage à base de titane, d'aluminium et de vanadium, à usage médical TA6V ELI.

Un autre but de l'invention est également de proposer un procédé de traitement de surface d'un matériau biocompatible, tel qu'un implant qui augmente le caractère hydrophile de sa surface.

### DESCRIPTION DETAILLEE

A cet effet, la présente invention concerne un procédé de traitement de surface d'un matériau métallique biocompatible, tel qu'un implant, selon la revendication 1.

Le principal avantage de ce procédé est d'utiliser pour l'étape dite de « sablage », c'est-à-dire l'étape de traitement mécanique abrasif, des grains constitués d'hydroxyapatite et de phosphate tricalcique, qui sont des matériaux constitutifs de la structure de l'os. Si des résidus restent présents à la surface, ceux-ci ne constituent pas des impuretés, mais peuvent au contraire participer à l'ostéo-intégration du matériau.

Jusqu'à présent un tel traitement mécanique au moyen de grains d'hydroxyapatite et de phosphate tricalcique n'avait été réalisé que pour des surfaces en matériau polymère (FR 2.906.147). Or, de manière surprenante, le traitement mécanique abrasif au moyen de grains de phosphate de calcium, tels qu'un mélange de grains d'hydroxy apatite et de phosphate tricalcique, effectué par projection sous haute pression desdits grains abrasifs, permet de créer, à la surface d'un matériau métallique, tel qu'un matériau en alliage de titane, des macroporosités en surface dudit matériau sous la forme d'alvéoles de dimensions de l'ordre de 50 µm à 250 µm.

Les traitements à chaud, à une température supérieure à 40°C, en milieux respectivement acide et sodique permettent ensuite de créer une microporosité (pores de l'ordre de 1 à 50 µm) ainsi qu'une nano-porosité (pores de taille inférieure au micromètre) dans lesdites premières alvéoles, de manière homogène sur l'ensemble de la surface traitée.

Le premier ancrage de l'implant s'effectue grâce à la macroporosité de surface du matériau ; la microporosité contribue à favoriser la création de ponts ou tentacules d'ancrage secondaire, et enfin la nano-porosité produit un effet de succion (capillarité) augmentant la vascularisation de ce greffon, et donc son ostéo-intégration.

De préférence, le matériau biocompatible est un alliage de titane, plus particulièrement un l'alliage à base de titane, d'aluminium et de vanadium, tel que l'alliage dénommé TA6V ELI (selon la norme ASTM F136).

Il a en effet été constaté, de manière surprenante, que la combinaison des étapes du procédé de traitement de surface, selon la présente invention, appliqué à l'alliage TA6V ELI permet de créer une surface très rugueuse et hydrophile sans faire apparaitre de cristaux de vanadium détachables ou laissé des résidus de matériaux abrasifs indésirables à la surface des implants.

Selon des caractéristiques avantageuses de l'invention :
Le mélange des grains d'hydroxyapatite et de phosphate tricalcique comprend de 80 à 90 % d'hydroxyapatite et de 10 à 20 % de phosphate tricalcique.

La dureté de ces grains est de préférence supérieure à 350 Hv (dureté Vickers)

Les grains abrasifs de phosphate de calcium, notamment d'hydroxyapatite et de phosphate tricalcique présentent une granulométrie comprise entre 160 et 400 micromètres, de préférence comprise entre 200 et 360 micromètres.

Le bain d'acide comprend de 45 à 55 % volumique d'acide sulfurique à 95% et de 45 à 55 % volumique d'acide chlorhydrique à 37%.

Le traitement acide est effectué par trempage dudit matériau dans un bain à une température comprise entre 60 et 70°C pendant une durée de 18 à 30 minutes, de préférence de 20 à 25 minutes.

Le traitement sodique est effectué par trempage dudit matériau dans de la soude à une concentration de 4 à 6 molaire et à une température de bain comprise entre 60 et 70°C pendant une durée comprise entre 18 et 30 minutes, de préférence entre 20 et 25 minutes.

Les traitements dans les bains acide et sodique sont avantageusement effectués sous agitation, et effectués immédiatement l'un à la suite de l'autre dans l'ordre indiqué ci-dessus.

La présente invention concerne également un implant selon la revendication 8.

L'implant selon l'invention ayant subi un traitement de surface au moyen du procédé décrit ci-dessus est également caractérisé en ce que l'angle de contact de la surface traitée est inférieur ou égal à 10° en présence d'eau distillée ou d'éthylène glycol en tant qu'agent mouillant. Ce caractère hydrophile de la surface augmente ainsi la capillarité dans les pores de la surface du matériau, en particulier pour les liquides physiologiques.

L'implant selon l'invention peut aussi être un implant en alliage de titane, d'aluminium et de vanadium, tel que l'alliage dénommé TA6V ELI, caractérisé en ce qu'il a subi un traitement de surface au moyen du procédé décrit ci-dessus, sa surface traitée présentant des teneurs réduites en aluminium et vanadium d'au moins 30 % par rapport à l'alliage de départ, mesurées par analyse EDS (Energy Dispersive Spectroscopy).

Le traitement de surface selon l'invention est adapté aux implants comportant une surface extérieure filetée.

### BREVE DESCRIPTION DES DESSINS

L'invention sera bien comprise à la lecture de la description suivante d'un exemple de réalisation, en référence aux dessins annexés, dans lesquels :
- la figure 1 est une image en microscopie électronique à balayage, avec un grossissement de 500, de la surface d'un implant traitée selon le procédé de la présente invention ;
- la figure 2 est une image de la surface de l'implant la figure 1 avec un grossissement de 2 000 ;
- la figure 3 est une image de la surface de l'implant la figure 1 avec un grossissement de 5 000 ;
- la figure 4 est une image de la surface de l'implant la figure 1 avec un grossissement de 10 000 ;
- la figure 5 est une image de la surface de l'implant la figure 1 avec un grossissement de 30 000 ;
- la figure 6 est un diagramme comparant les angles de contact des surfaces d'implant des exemples 1 et 2 mesurées avec différents agents mouillants.

### EXEMPLES

### Exemple 1 selon l'invention

Le traitement de surface d'un implant en alliage TA6V ELI (selon la norme ASTM F136 alliage à base de titane, renfermant 6 % massique d'aluminium et 4 % massique de vanadium : voir tableau 1) est effectué selon un traitement dénommé « Nano-mordançage » en trois étapes consécutives, détaillées ci-après.

**Tableau 1**

| Fe % max. | O % max. | N % max. | C % max. | H % max. | Al % | V % |
|---|---|---|---|---|---|---|
| 0,25 | 0,13 | 0,05 | 0,08 | 0,012 | 5,50 - 6,50 | 3,50 - 4,50 |

### Étape 1 : Traitement mécanique

Les implants sont sablés avec un abrasif composé d'hydroxyaptite (85 ±5 %) et de phosphate tricalcique (15 ± 5 %) de dureté Vickers égale à 532 Hv, avec un diamètre des grains de la poudre compris entre 160 et 400 µm, avec une prédominance des grains de taille entre 200 et 360 µm de diamètre.

Cette étape consiste à créer des porosités de taille allant jusqu'à 250 µm de diamètre. La projection des grains d'abrasifs est réalisée au moyen d'une buse disposée à environ 10 à 20 cm de la surface de l'implant sous une pression de 5 à 7 bar pendant 60 ± 10 secondes.

### Étape 2 : Traitement acide

Cette étape consiste à créer des porosités de quelques dizaines de microns de diamètre et de profondeur de façon homogène sur toute la surface traitée.

Le traitement est réalisé dans un mélange composé de deux acides. La composition d'acide et les paramètres de traitement sont décrits ci-dessous :
- Composition du bain : 50% ± 5% en volume d'acide sulfurique à 95% et 50% ± 5% en volume d'acide chlorhydrique à 37%.
- Température de traitement : 67 ± 2,5 °C,
- Durée du traitement : 22 ± 1 min,
- Sous agitation du bain.

Une durée de traitement supérieure ou une température au-dessus de la gamme indiquée conduit à une attaque de la macro-rugosité créée à l'étape 1.

### Étape 3 : Traitement sodique

Ce traitement a pour but de créer un tissu de porosité de taille nanométrique à la surface de l'implant.

Cette étape est réalisée dans un bain d'hydroxyde de sodium, comme suit :
- Composition du bain : hydroxyde de sodium à 5 ± 0,5 M (5 ± 0,5 mol/L)
- Température du bain : 67 ± 2.5 °C,
- Durée du traitement : 22 ± 1 min,
- Agitation du bain.

Un traitement sodique insuffisant (concentration en NaOH insuffisante, pores inférieurs à 4 mol/L, température plus faible ou durée de traitement plus courte) conduit à des surfaces moins hydrophiles avec des angles de contact en présence d'eau distillée, d'éthylène glycol ou de diiodométhane supérieurs à 50°, voire supérieurs à 70°, ainsi qu'à une nano-porosité insuffisante.

### Résultats des observations MEB :

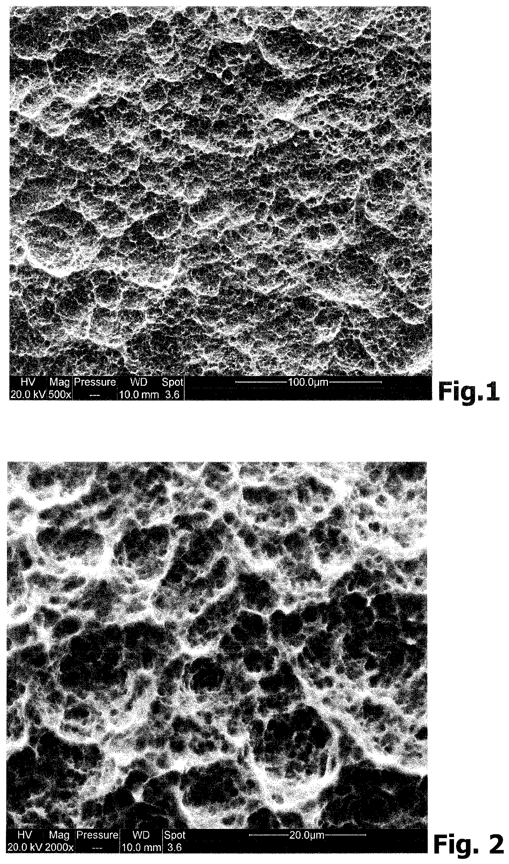

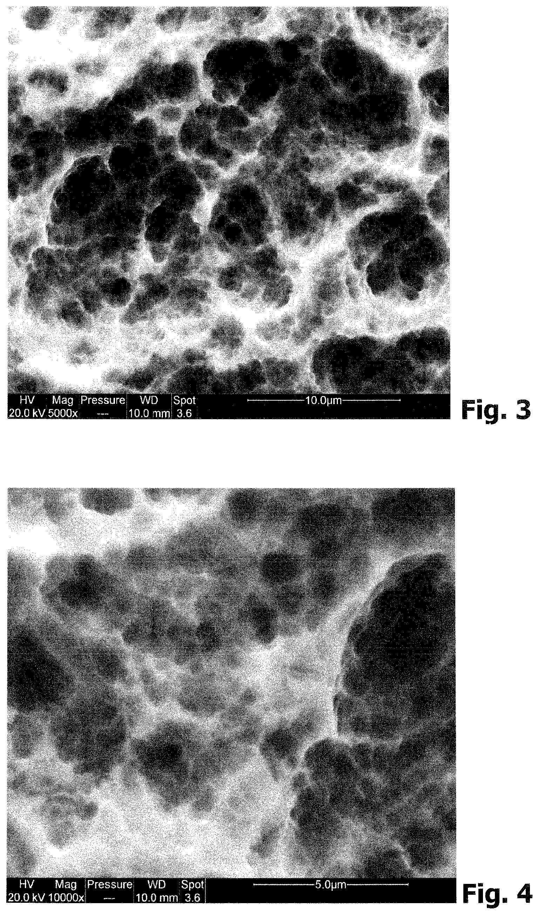

Les images obtenues sous observation au microscope électronique à balayage, présentées sur les figures 1 à 5 sous différents grossissements (respectivement 500, 2000, 5000, 10 000, et 30 000), montrent une surface très accidentée et très rugueuse.

La surface présente, en effet, un aspect avec des porosités de quelques dizaines de microns de diamètre qui elles-mêmes comportent des porosités de quelques microns de diamètre et de profondeur. Ces mêmes microporosités comportent également des porosités de diamètre et de profondeur inférieures au micron, à savoir quelques centaines de nanomètres. La présence de ce triple niveau de porosité à la surface du matériau constitue un avantage important pour l'ostéointégration de l'implant.

Nous pouvons même observer sur l'image MEB avec un grossissement 30 000 un tissu de fibres très fines qui recouvre toute la surface traitée.

Les analyses EDS (Energy Dispersive Spectroscopy, réalisées sous vide avec un appareil FEl QUANTA 200) de cette surface montrent une forte présence de titane et d'oxygène (donc probablement d'oxyde de titane), comme présenté dans le tableau 2 ci-après qui compare la composition chimique de la surface traitée et celle de l'alliage brut avant traitement.

**Tableau 2**

| Éléments | Composition massique (%) | |
|---|---|---|
| | Alliage à surface traitée | Alliage brut |
| Titane | 66,15 | 86,95 - 89,20 |
| Oxygène | 26,11 | 0,5 |
| Aluminium | 3,50 | 5,5 - 6,75 |
| Vanadium | 1,92 | 3,5 - 4,5 |
| Carbone | 1,44 | 0,08 |
| Sodium | 0,11 | - |
| Fer | - | 0,4 |
| Hydrogène | - | 0,015 |

La surface de l'alliage traitée selon l'invention présente des teneurs en aluminium et en vanadium plus basses que celles présentes à la surface d'un alliage de titane grade 23 (TA6V ELI) brut. Ces analyses mettent également en évidence la forte présence d'oxygène à la surface de l'implant traité, ce qui signifie qu'il y a formation d'une couche d'oxyde de titane.

### Exemple 2 comparatif

Un traitement de surface d'un implant en alliage à base de titane Straumann SLA^{®} est effectué selon les mêmes trois étapes et dans des conditions identiques à celles de l'exemple 1 ci-dessus.

### Mesures de rugosité :

Les résultats des mesures de rugosité Ra et Rz (effectuées au moyen d'un appareil MITOTOYO, référence SJ400) comparant les surfaces des implants traités selon l'exemple 1 et l'exemple comparatif 2 sont présentés dans le tableau 3 ci-dessous.

Les significations de Ra et Rz sont les suivantes :
« Ra » : écart moyen. C'est la moyenne arithmétique des valeurs absolues des écarts, entre les pics et les creux. « Ra » mesure la distance entre cette moyenne et la « ligne centrale ».
« Rz » : régularité. C'est la moyenne de la dénivellation la plus importante entre le plus haut sommet d'un pic et le fond le plus bas d'un creux, observés sur 5 longueurs.

**Tableau 3**

| Surfaces testées | Ra | Rz | Commentaires |
|---|---|---|---|
| Exemple 1 | 1,90 µm | 10,46 µm | La surface de l'implant de l'exemple 1 est plus rugueuse que celle de l'implant de l'exemple 2 |
| Exemple 2 (Comp.) | 1,83 µm | 10,03 µm | |

### Mesures d'angle de contact :

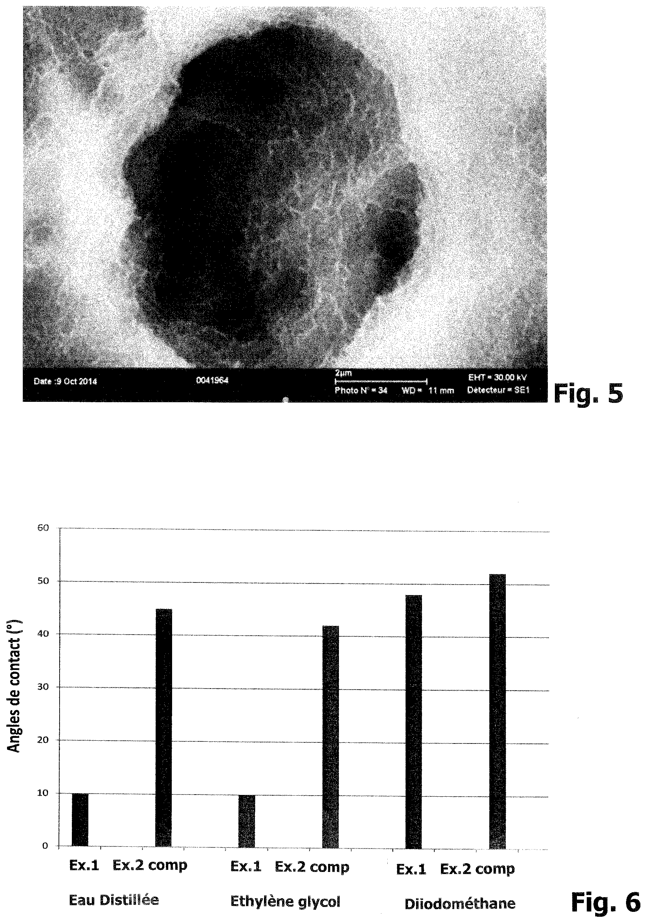

Des mesures d'angle de contact ont été réalisées avec trois liquides différents (eau distillée, éthylène glycol et diiodométhane) pour les implants des exemples 1 et 2. Les résultats, présentés sur la figure 6, montrent des différences significatives lorsque l'agent mouillant est, soit l'eau distillée, soit l'éthylène glycol, prouvant un caractère hydrophile bien supérieur pour la surface traitée selon le procédé de la présente invention.

## Revendications

1. Procédé de traitement de surface d'un matériau métallique biocompatible en alliage de titane, tel qu'un implant, comprenant les étapes consécutives suivantes :
i) traitement mécanique abrasif de la surface dudit matériau au moyen de grains abrasifs à base d'un mélange d'hydroxyapatite et de phosphate tricalcique ;
ii) traitement à l'acide par trempage, à une température supérieure à 40°C, dudit matériau dans un bain comprenant de l'acide sulfurique et de l'acide chlorhydrique, suivi d'au moins un rinçage, de préférence deux rinçages, à l'eau déminéralisée ;
iii) traitement sodique par trempage, à une température supérieure à 40°C, dudit matériau dans un bain à base de soude suivi d'au moins un rinçage, de préférence deux rinçages, à l'eau déminéralisée et d'un séchage à l'air chaud
lesdites étapes permettant de créer, à la surface dudit matériau métallique biocompatible une macroporosité sous la forme d'alvéoles de diamètre de l'ordre de 50 µm à 250 µm, lesdites alvéoles comportant des pores de diamètre et de profondeur de 1 µm à 50 µm, et des pores de diamètre et de profondeur inférieurs au micromètre, de manière homogène sur l'ensemble de la surface traitée, ladite surface traitée présentant une rugosité de surface Ra supérieure ou égale à 1,90 µm.

2. Procédé selon la revendication 1,
**caractérisé en ce que** le matériau métallique biocompatible est un alliage de titane, d'aluminium et de vanadium, de préférence l'alliage de titane renfermant de 5,50 à 6,50 % massique d'aluminium de 3,50 à 4,50 % massique de vanadium, 0,25 % max. de Fer, 0,13 % max. d'Oxygène, 0,05 % max. d'azote , 0,08 % max. de Carbone et 0,012 % max. d'Hydrogène

3. Procédé selon l'une quelconque des revendications précédentes,
**caractérisé en ce que** le mélange des grains d'hydroxyapatite et de phosphate tricalcique comprend de 80 à 90 % d'hydroxyapatite et de 10 à 20 % de phosphate tricalcique.

4. Procédé selon la revendication 3,
**caractérisé en ce que** les grains abrasifs d'hydroxyapatite et de phosphate tricalcique présentent une granulométrie comprise entre 160 et 400 micromètres, de préférence comprise entre 200 et 360 micromètres.

5. Procédé selon l'une quelconque des revendications précédentes,
**caractérisé en ce que** le bain d'acide comprend de 45 à 55 % volumique d'acide sulfurique à 95% et de 45 à 55 % volumique d'acide chlorhydrique à 37%.

6. Procédé selon la revendication 5,
**caractérisé en ce que** le traitement acide est effectué par trempage dudit matériau dans un bain à une température comprise entre 60 et 70°C pendant une durée de 18 à 30 minutes, de préférence de 20 à 25 minutes.

7. Procédé selon l'une quelconque des revendications précédentes,
**caractérisé en ce que** le traitement sodique est effectué par trempage dudit matériau dans de la soude à une concentration de 4 à 6 molaire et à une température de bain comprise entre 60 et 70°C pendant une durée comprise entre 18 et 30 minutes, de préférence entre 20 et 25 minutes.

8. Implant en matériau métallique biocompatible en alliage de titane ayant subi un traitement de surface au moyen du procédé selon l'une quelconque des revendications précédentes,
**caractérisé en ce que** sa surface présente une macroporosité sous la forme d'alvéoles de diamètre de l'ordre de 50 µm à 250 µm, lesdites alvéoles comportant des pores de diamètre et de profondeur de 1 µm à 50 µm, et des pores de diamètre et de profondeur inférieurs au micromètre, de manière homogène sur l'ensemble de la surface traitée, ladite surface traitée présentant une rugosité de surface Ra supérieure ou égale à 1,90 µm.

9. Implant selon la revendication 8,
**caractérisé en ce que** l'angle de contact de la surface traitée est inférieur ou égal à 10° en présence d'eau distillée ou d'éthylène glycol en tant qu'agent mouillant.

10. Implant selon la revendication 8 ou 9, en alliage de titane, d'aluminium et de vanadium,
**caractérisé en ce qu'**il a subi un traitement de surface au moyen du procédé selon l'une des revendications 2 à 7, sa surface traitée présentant des teneurs réduites en aluminium et vanadium d'au moins 30 % par rapport à l'alliage de départ, mesurées par analyse EDS réalisée sous vide, et la présence d'oxyde de titane.

11. Implant selon la revendication 10,
**caractérisé en ce qu'**il est en alliage de titane renfermant de 5,50 à 6,50 % massique d'aluminium, de 3,50 à 4,50 % massique de vanadium, 0,25 % max. de Fer, 0,13 % max. d'Oxygène, 0,05 % max. d'azote, 0,08 % max. de Carbone et 0,012 % max. d'Hydrogène.

## Patentansprüche

1. Verfahren zur Oberflächenbehandlung eines biokompatiblen Metallmaterials aus einer Titanlegierung, wie eines Implantats, das die folgenden aufeinanderfolgenden Schritte umfasst:
i) eine abrasive mechanische Behandlung der Oberfläche des Materials mit Schleifkörnern auf der Basis einer Mischung von Hydroxyapatit und Tricalciumphosphat;
ii) eine Säurebehandlung bei einer Temperatur über 40 °C durch Tauchen des Materials in ein Schwefelsäure und Salzsäure enthaltendes Bad, gefolgt von mindestens einem Spülvorgang, vorzugsweise zwei Spülvorgängen, mit entmineralisiertem Wasser;
iii) eine Natriumbehandlung bei einer Temperatur über 40 °C durch Tauchen des Materials in ein Bad auf Natriumhydroxid-Basis, gefolgt von mindestens einem Spülvorgang, vorzugsweise zwei Spülvorgängen, mit entmineralisiertem Wasser und einem Trocknen mit heißer Luft;
wobei die Schritte die Bildung einer Makroporosität in Form von Vertiefungen mit einem Durchmesser in der Größenordnung von 50 µm bis 250 µm auf der Oberfläche des biokompatiblen Metallmaterials, wobei die Vertiefungen Poren mit einem Durchmesser und einer Tiefe von 1 µm bis 50 µm und Poren mit einem Durchmesser und einer Tiefe von weniger als einem Mikrometer homogen über die gesamte behandelte Oberfläche aufweisen, wobei die behandelte Oberfläche eine Oberflächenrauheit Ra von mehr als oder gleich 1,90 µm aufweist.

2. Verfahren nach Anspruch 1,
**dadurch gekennzeichnet, dass** es sich bei dem biokompatiblen Metallmaterial um eine Legierung von Titan, Aluminium und Vanadium, vorzugsweise eine Titanlegierung, handelt, die 5,50 bis 6,50 Gew.-% Aluminium, 3,50 bis 4,50 Gew.-% Vanadium, max. 0,25 % Eisen, max. 0,13 % Sauerstoff, max. 0,05 % Stickstoff, max. 0,08 % Kohlenstoff und max. 0,012 % Wasserstoff enthält.

3. Verfahren nach einem der vorhergehenden Ansprüche, **dadurch gekennzeichnet, dass** die Mischung von Hydroxyapatit- und Tricalciumphosphat-Körnern 80 bis 90 % Hydroxyapatit und 10 bis 20 % Tricalciumphosphat umfasst.

4. Verfahren nach Anspruch 3,
**dadurch gekennzeichnet, dass** die Hydroxyapatit- und Tricalciumphosphat-Schleifkörner eine Körnung zwischen 160 und 400 Mikrometer, vorzugsweise zwischen 200 und 360 Mikrometer, aufweisen.

5. Verfahren nach einem der vorhergehenden Ansprüche, **dadurch gekennzeichnet, dass** das Säurebad 45 bis 55 Vol.-% 95%ige Schwefelsäure und 45 bis 55 Vol.-% 37%ige Salzsäure umfasst.

6. Verfahren nach Anspruch 5,
**dadurch gekennzeichnet, dass** die Säurebehandlung durch Tauchen des Materials in ein Bad mit einer Temperatur zwischen 60 und 70 °C für eine Dauer von 18 bis 30 Minuten, vorzugsweise von 20 bis 25 Minuten, erfolgt.

7. Verfahren nach einem der vorhergehenden Ansprüche, **dadurch gekennzeichnet, dass** die Natriumbehandlung durch Tauchen des Materials in Natriumhydroxid mit einer Konzentration von 4 bis 6 Mol und bei einer Badtemperatur zwischen 60 und 70 °C für eine Dauer zwischen 18 und 30 Minuten, vorzugsweise zwischen 20 und 25 Minuten, erfolgt.

8. Implantat aus einem biokompatiblen Metallmaterial aus einer Titanlegierung, das einer Oberflächenbehandlung mittels des Verfahrens nach einem der vorhergehenden Ansprüche unterzogen worden ist, **dadurch gekennzeichnet, dass** seine Oberfläche eine Makroporosität in Form von Vertiefungen mit einem Durchmesser in der Größenordnung von 50 µm bis 250 µm aufweist, wobei die Vertiefungen Poren mit einem Durchmesser und einer Tiefe von 1 µm bis 50 µm und Poren mit einem Durchmesser und einer Tiefe von weniger als einem Mikrometer homogen über die gesamte behandelte Oberfläche aufweisen, wobei die behandelte Oberfläche eine Oberflächenrauheit Ra von mehr als oder gleich 1,90 µm aufweist.

9. Implantat nach Anspruch 8,
**dadurch gekennzeichnet, dass** der Kontaktwinkel der behandelten Oberfläche in Gegenwart von destilliertem Wasser oder Ethylenglycol als Benetzungsmittel kleiner als oder gleich 10° ist.

10. Implantat nach Anspruch 8 oder 9 aus einer Legierung von Titan, Aluminium und Vanadium,
**dadurch gekennzeichnet, dass** es einer Oberflächenbehandlung mittels des Verfahrens nach einem der Ansprüche 2 bis 7 unterzogen worden ist, seine behandelte Oberfläche Aluminium- und Vanadiumgehalte, die in Bezug auf die Ausgangslegierung um mindestens 30 % vermindert sind, gemessen mittels einer im Vakuum durchgeführten EDS-Analyse, und das Vorhandensein von Titanoxid aufweist.

11. Implantat nach Anspruch 10,
**dadurch gekennzeichnet, dass** es aus einer Titanlegierung besteht, die 5,50 bis 6,50 Gew.-% Aluminium, 3,50 bis 4,50 Gew.-% Vanadium, max. 0,25 % Eisen, max. 0,13 % Sauerstoff, max. 0,05 % Stickstoff, max. 0,08 % Kohlenstoff und max. 0,012 % Wasserstoff enthält.

## Claims

1. Process for the surface treatment of a titanium alloy biocompatible metallic material, such as an implant, comprising the following consecutive steps:
i) abrasive mechanical treatment of the surface of said material by means of abrasive grains based on a mixture of hydroxyapatite and tricalcium phosphate;
ii) acid treatment by soaking said material, at a temperature above 40°C, in a bath comprising sulfuric acid and hydrochloric acid, followed by at least one rinse, preferably two rinses, with demineralized water;
iii) sodium treatment by soaking said material, at a temperature above 40°C, in a sodium hydroxide-based bath followed by at least one rinse, preferably two rinses, with demineralized water and by hot air drying,
said steps making it possible to create, on the surface of said biocompatible metallic material, a macroporosity in the form of cells with a diameter of the order of 50 µm to 250 µm, said cells comprising pores with a diameter and depth of 1 µm to 50 µm, and pores with a diameter and depth of less than one micrometre, homogeneously over the entire surface treated, said treated surface having a surface roughness Ra of greater than or equal to 1.90 µm.

2. Process according to Claim 1,
**characterized in that** the biocompatible metallic material is an alloy of titanium, aluminium and vanadium, preferably the titanium alloy containing from 5.50% to 6.50% by weight of aluminium, from 3.50% to 4.50% by weight of vanadium, 0.25% max. of iron, 0.13% max. of oxygen, 0.05% max. of nitrogen, 0.08% max. of carbon and 0.012% max. of hydrogen.

3. Process according to either one of the preceding claims,
**characterized in that** the mixture of hydroxyapatite and tricalcium phosphate grains comprises from 80% to 90% of hydroxyapatite and from 10% to 20% of tricalcium phosphate.

4. Process according to Claim 3,
**characterized in that** the abrasive grains of hydroxyapatite and tricalcium phosphate have a particle size of between 160 and 400 micrometres, preferably between 200 and 360 micrometres.

5. Process according to any one of the preceding claims,
**characterized in that** the acid bath comprises from 45% to 55% by volume of 95% sulfuric acid and from 45% to 55% by volume of 37% hydrochloric acid.

6. Process according to Claim 5,
**characterized in that** the acid treatment is carried out by soaking said material in a bath at a temperature between 60°C and 70°C for a duration of from 18 to 30 minutes, preferably from 20 to 25 minutes.

7. Process according to any one of the preceding claims,
**characterized in that** the sodium treatment is carried out by soaking said material in sodium hydroxide at a molar concentration of 4 to 6 M and at a bath temperature of between 60°C and 70°C for a duration of between 18 and 30 minutes, preferably between 20 and 25 minutes.

8. Implant made of titanium alloy biocompatible metallic material that has undergone a surface treatment by means of the process according to any one of the preceding claims,
**characterized in that** its surface has a macroporosity in the form of cells with a diameter of the order of 50 µm to 250 µm, said cells comprising pores with a diameter and depth of 1 µm to 50 µm, and pores with a diameter and depth of less than one micrometre, homogeneously over the entire surface treated, said treated surface having a surface roughness Ra of greater than or equal to 1.90 µm.

9. Implant according to Claim 8,
**characterized in that** the contact angle of the treated surface is less than or equal to 10° in the presence of distilled water or ethylene glycol as wetting agent.

10. Implant according to Claim 8 or 9, made of an alloy of titanium, aluminium and vanadium,
**characterized in that** it has undergone a surface treatment by means of the process according to one of Claims 2 to 7, its treated surface having contents of aluminium and vanadium that are reduced by at least 30% relative to the initial alloy, measured by EDS analysis carried out under vacuum, and the presence of titanium oxide.

11. Implant according to Claim 10,
**characterized in that** it is made of titanium alloy containing from 5.50% to 6.50% by weight of aluminium, from 3.50% to 4.50% by weight of vanadium, 0.25% max. of iron, 0.13% max. of oxygen, 0.05% max. of nitrogen, 0.08% max. of carbon and 0.012% max. of hydrogen.
